(19) **Europäisches Patentamt European Patent Office Office européen des brevets**

(11) **EP 4 623 989 A1**

(12) **EUROPEAN PATENT APPLICATION**
published in accordance with Art. 153(4) EPC

(43) Date of publication:
**01.10.2025 Bulletin 2025/40**

(21) Application number: **23896736.8**

(22) Date of filing: **27.11.2023**

(51) International Patent Classification (IPC):
**A61N 5/10** (2006.01)

(52) Cooperative Patent Classification (CPC):
**A61N 5/10; G06F 30/25; G16H 20/40; G16H 40/60**

(86) International application number:
**PCT/CN2023/134368**

(87) International publication number:
**WO 2024/114585 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.11.2022 CN 202211500417**
**24.11.2023 CN 202311580219**

(71) Applicant: **Neuboron Therapy System Ltd.**
**Xiamen, Fujian 361026 (CN)**

(72) Inventors:
• **ZHONG, Wanbing**
**Nanjing, Jiangsu 211112 (CN)**
• **CHEN, Jiang**
**Nanjing, Jiangsu 211112 (CN)**

(74) Representative: **Gunzelmann, Rainer**
**Wuesthoff & Wuesthoff**
**Patentanwälte und Rechtsanwalt PartG mbB**
**Schweigerstraße 2**
**81541 München (DE)**

(54) **OPTIMIZATION METHOD FOR MONTE CARLO CALCULATION AND NEUTRON CAPTURE THERAPY SYSTEM**

(57) An optimization method for Monte Carlo calculation and a neutron capture therapy system. The method at least comprises: simulating a particle transport process using a transport calculation method; statistically determining expected dose values of particles using a dose counting method; and calculating an average expected dose value of a voxel grid on the basis of the expected dose value of each of the particles. By means of optimizing the simulation of the particle transport process or the dose counting method, the Monte Carlo calculation is optimized, thus shortening the therapy planning time.

```
┌────────────────────────────────────────┐
│  Simulate a transport process of each of │ ──── S10
│              particles                   │
└────────────────────────────────────────┘
                    │
                    ▼
┌────────────────────────────────────────┐
│  Count an expected dose value of the     │ ──── S11
│              particle                    │
└────────────────────────────────────────┘
                    │
                    ▼
┌────────────────────────────────────────┐
│  Calculate a mean expected dose value of │ ──── S12
│          each of voxel grids             │
└────────────────────────────────────────┘
```

**FIG. 1**

EP 4 623 989 A1

**Description**

**TECHNICAL FIELD**

The present disclosure relates to a radiotherapy system and method, and in particular to a method for optimizing Monte Carlo calculation and a neutron capture therapy system.

**BACKGROUND**

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. For example, with specific aggregation of boron-containing drugs in tumor cells, and in cooperation with accurate neutron beam control, boron neutron capture therapy (BNCT) in neutron capture therapy serves as a better alternative to treat the cancers.

According to the BNCT, with a large capture cross section of the boron ($^{10}$B)-containing drug for thermal neutrons, and through the $^{10}$B(n,$\alpha$)$^{7}$Li neutron capture reaction and the nuclear fission reaction, $^{4}$He and $^{7}$Li heavy charged particles are generated. The total range of the two particles is approximately equivalent to a cell size, such that the radiation damage to an organism is limited to a cell level. When the boron-containing drug is aggregated to the tumor cells selectively, and an appropriate neutron source is provided, a purpose of locally killing the tumor cells on premise of no serious damage to normal tissues can be achieved.

In order that radiating particles kill more tumor cells and cause less damages to the normal cells, an image of the patient is scanned through computed tomography (CT) or positron emission tomography (PET) before treatment. Tissue information of the body is acquired according to a scanned result. A calculation model is established according to the tissue information and a radiation source to simulate transport processes of the radiating particles in the body, thereby obtaining a dose distribution of the radiating particles in the body. Then, a scheme with the most optimal dose distribution in the patient is selected for treatment.

At present, the dose calculation module in the treatment planning system is mainly configured to simulate the radiating particles with a Monte Carlo method. Compared with the conventional radiotherapy in which the movement processes of photons and electrons are to be simulated, and the BNCT in which the movement processes of neutrons and photons are to be simulated, the Monte Carlo method can establish the model accurately and simulate the random walk processes of the radiating particles in the body truly. However, the Monte Carlo method is restricted by the slow convergence speed and the long calculation time. Therefore, it is desired to optimize and accelerate dose calculation of the Monte Carlo method, to obtain the dose distribution more quickly.

**SUMMARY**

In view of the defects in the prior art, an aspect of the present disclosure provides a method for optimizing Monte Carlo calculation, at least including: simulating a transport process of a particle with a transport calculation method; counting an expected dose value of the particle with a dose counting method; and calculating a mean expected dose value of each of voxel grid on the basis of the expected dose value of the particle. By optimally simulating the transport process or the dose counting method of the particle, the Monte Carlo calculation is optimized, thereby shortening time for formulating a treatment plan.

The transport calculation method at least includes: acquiring information of the particle; calculating a macroscopic cross-section according to the information of the particle; acquiring a next transport point or a collision point of the particle on the basis of the macroscopic cross-section; and determining a survival status of the particle. Further, the acquiring a next transport point or a collision point of the particle on the basis of the macroscopic cross-section includes: sampling the next transport point or the collision point of the particle, and updating the information of the particle.

Further, the determining a survival status of the particle at least includes: if the particle is in a survival state, determining whether to recalculate the macroscopic cross-section; and if the particle is not in the survival state, stopping simulating the transport process of the particle.

The information of the particle at least includes: position information, direction information, and energy information of the particle.

Further, the method for optimizing Monte Carlo calculation further includes: determining a material of a voxel grid of the particle on the basis of the position information and the direction information of the particle, and calculating the macroscopic cross-section on the basis of the energy information of the particle and the material of the voxel grid of the particle.

Further, the determining whether to recalculate the macroscopic cross-section at least includes: determining whether the energy of the particle changes; determining whether the material of the voxel grid of the particle changes; if the energy of the particle or the material of the voxel grid of the particle changes, recalculating the macroscopic cross-section according to the energy of the particle and the material of the voxel grid of the particle; and if the energy of the particle and the material of the voxel grid of the particle do not change, acquiring the next transport point or the collision point of the particle on the basis of the macroscopic cross-section. When both the material of the voxel grid of the particle and the energy of the particle do not change, the macroscopic cross-section in the previous step can be used, and the next transport point or the collision point of the particle is acquired directly on the basis of the macroscopic cross-section. This reduces a number of times for calculating the macroscopic cross-section, thereby reducing the operation time.

The dose counting method at least includes: counting a track length of the particle in the voxel grid; calculating the expected dose value of the particle in the voxel on the basis of the track length of the particle and a corresponding macroscopic cross-section; and calculating the mean expected dose value of the voxel grid.

Further, the calculating the mean expected dose value of the voxel grid includes: calculating the mean expected dose value of the voxel grid by:

$$mean = \frac{\sum_{i=1}^{N} t_i}{N} \qquad (1)$$

where, i is a serial number of the particle, $t_i$ is an expected dose value of the particle i in the voxel grid, and N is a total number of particles.

Further, the calculating the mean expected dose value of the voxel grid further includes: when the particle passes twice through the voxel grid, counting the expected dose value of the particle twice, and keeping the total number N of particles unchanged. By omitting the process for merging the expected dose values of the same particle passing twice through the voxel grid, directly taking two individual particles for calculation, and keeping the total number N of particles unchanged, the method ensures the accuracy of the calculated dose result, and reduces the time for the dose calculation, thereby reducing the operation time. Further, the dose counting method further at least includes: calculating a dose error of the voxel grid.

Further, the calculating a dose error of the voxel grid includes: calculating the dose error of the voxel grid by:

$$error = \sqrt{\frac{\frac{\sum_{i=1}^{N} t_i^2}{N} - mean^2}{N-1}} \qquad (2)$$

where, i is a serial number of the particle, $t_i$ is an expected dose value of the particle i in the voxel grid, and N is a total number of particles.

Further, the calculating the dose error of the voxel grid further includes: when the particle passes twice through the voxel grid, counting the expected dose value of the particle twice, and keeping the total number N of particles.

Further, the particles include a neutron and a photon.

Another aspect of the present disclosure provides a neutron capture therapy system, including: an image acquisition module configured to acquire a medical image of an irradiated body; and a treatment planning module configured to establish a three-dimensional (3D) voxel model on the basis of the medical image and formulate a treatment plan, where the treatment planning module includes at least a transport calculation unit and a dose counting unit, the transport calculation unit is configured to simulate a transport process of a particle, and the dose counting unit is configured to count an expected dose value of the particle, and calculate a mean expected dose value of each of voxel grids on the basis of the expected dose value of the particle.

Further, the neutron capture therapy system further includes a dose calculation unit; the dose calculation unit is configured to calculate a tissue dose value on the basis of the mean expected dose value of the voxel grid; and the treatment planning module is further configured to formulate the treatment plan on the basis of the tissue dose value.

According to the neutron capture therapy system provided by the present disclosure, by optimally simulating the transport process or the dose counting method of the particle, the Monte Carlo calculation is optimized, thereby shortening time for formulating the treatment plan.

## BRIEF DESCRIPTION OF THE DRAWINGS

FIG. 1 is a flowchart of Monte Carlo calculation according to the present disclosure;
FIG. 2 is a flowchart for simulating a transport process of a particle according to the present disclosure;
FIG. 3 is a flowchart for counting an expected dose value of a particle according to the present disclosure; and
FIG. 4 is a flowchart for specifically counting an expected dose value of a particle according to the present disclosure.

## DETAILED DESCRIPTION OF THE EMBODIMENTS

Embodiments of the present disclosure will be further described in detail below with reference to the accompanying drawings, such that those skilled in the art can implement the present disclosure with reference to the description.

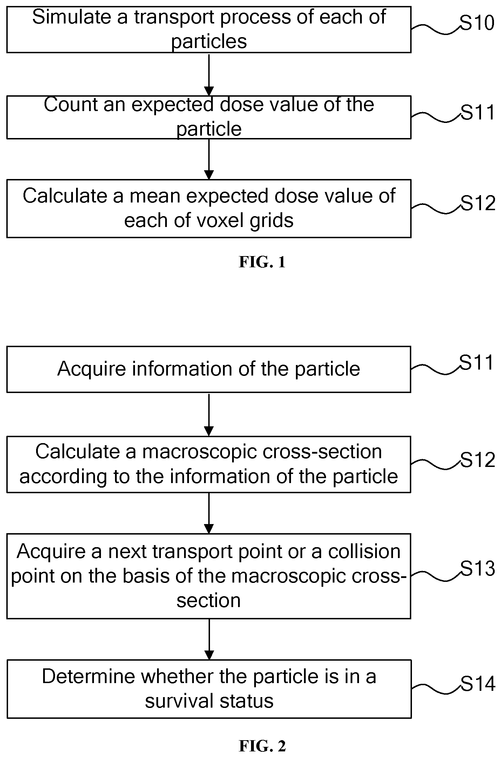

At present, radiotherapy is commonly used to treat cancers. Neutron capture therapy, particularly BNCT, has been applied increasingly in recent years as an effective method to treat the cancers. Neutrons in the BNCT can be supplied by a nuclear reactor or an accelerator. According to the BNCT, with a large capture cross section of the boron ($^{10}$B)-containing drug for thermal neutrons, and through the $^{10}$B(n,$\alpha$)$^7$Li neutron capture reaction and the nuclear fission reaction, $^4$He and $^7$Li heavy charged particles are generated. FIG. 1 and FIG. 2 respectively illustrate a schematic view of a boron neutron capture reaction and an equation of a nuclear reaction in $^{10}$B(n,$\alpha$)$^7$Li neutron capture. The two heavy charged particles have an average energy of about 2.33 MeV, and have characteristics of the high LET, and the short range. The alpha particle has the LET of 150 keV/$\mu$m, and the range of 8 $\mu$m. The $^7$Li heavy charged particle has the LET of 175 keV/$\mu$m, and the range of 5 $\mu$m. The total range of the two particles are approximately equivalent to a cell size, such that the radiation damage to an organism is limited to a cell level. When the boron-containing drug is gathered to the tumor cells selectively, and an appropriate neutron source is provided, a purpose of locally killing the tumor cells on premise of no serious damage to normal tissues can be achieved.

No matter whether a neutron source in the BNCT comes from the nuclear reactor or the nuclear reaction between the charged particles and the target, a mixed radiation field is generated as a matter of fact, that is, the generated beam includes neutrons and photons having energies from low to high. As for the BNCT on deep tumors, except the epithermal neutrons, the higher the content of remaining radioactive rays, the greater the proportion causing non-selective dose deposition in the normal tissue. Therefore, the radioactive rays causing the unnecessary dose deposition should be reduced as much as possible. For better understanding on a dose distribution of neutrons in the body, except the air beam quality, a head prosthesis is used to calculate the dose distribution, and the prosthesis beam quality serves as a reference to design the neutron beam in the embodiment of the present disclosure.

For neutron sources in the clinical BNCT, the International Atomic Energy Agency (IAEA) provides five suggestions for the air beam quality. The five suggestions can be used to compare advantages and disadvantages of different neutron sources, and used as a reference basis to select a neutron generation method and design a beam shaper. The five suggestions are as follows:

Epithermal neutron flux> 1*10$^9$ n/cm$^2$s
Fast neutron contamination < 2*10$^{-13}$ Gy-cm$^2$/n
Photon contamination < 2*10$^{-13}$ Gy-cm$^2$/n
Thermal to epithermal neutron flux ratio < 0.05
Epithermal neutron current to flux ratio > 0.7

Note: The epithermal neutrons have an energy range between 0.5e V to 40 keV, the thermal neutrons have an energy range of less than 0.5 eV, and the fast neutrons have an energy range of greater than 40 keV.

In order to kill more tumor cells, and reduce the damage of radioactive rays on normal tissues, it is very crucial to formulate a treatment plan in the neutron capture therapy. At present, the Monte Carlo method for formulating the treatment plan is restricted for the slow convergence speed and the low calculation time. Therefore, the present disclosure provides a method for optimizing Monte Carlo calculation to improve the calculation speed. The present disclosure is described in detail below with reference to the specific embodiments:

As shown in FIG. 1, an aspect of the present disclosure provides a method for optimizing Monte Carlo calculation to improve a calculation speed. The method for optimizing Monte Carlo calculation at least includes: A transport process of a particle is simulated with a transport calculation method. An expected dose value of the particle is counted with a dose counting method. A mean expected dose value of each of voxel grids is calculated on the basis of the expected dose value of the particle. By optimally simulating the transport process or the dose counting method of the particle, the dose calculation method is optimized, thereby shortening time for formulating a treatment plan. The Monte Carlo method for calculating the dose is intended to simulate a random movement of each radiating particle in the body or medium, and count a dose of the particle in the movement. In the process for simulating the movement of the particle with the conventional Monte Carlo method, an initial attribute of a source particle is randomly sampled. A macroscopic cross-section of the particle in a medium is calculated, so as to calculate a collision probability. A next transport point or a collision point is calculated on the basis of the macroscopic cross-section. If the particle dies upon collision (for example, the particle is absorbed or cut off), the calculation is stopped. That is, simulating the movement of the particle is stopped. If the particle is still survival, the above steps are repeated. In order to ensure the accuracy of dose calculation, the voxel model used for the dose calculation features a large number of grids (such as millions of grids) with a small size (such as 1 mm). Hence, same grids are very likely to be filled with a same material. When the energy of the particle is unchanged, and the particle moves to the adjacent grid of the same material, the calculated macroscopic cross-section is the same.

As shown in FIG. 2, in the method for optimizing Monte Carlo calculation provided by the present disclosure, the transport calculation method at least includes: Information of the particle is acquired.

A macroscopic cross-section is calculated according to the information of the particle.

A next transport point or a collision point of the particle is acquired on the basis of the macroscopic cross-section.

A survival status of the particle is determined. For the step that the information of the particle is acquired:

Each radiating particle moves randomly in the body or medium. To simulate the transport process of the particle, the information of the particle is to be acquired. The information of the particle at least includes a position, a direction, and an energy. In an optional implementation, neutrons and photons are radiant to the body in treatment. When the treatment plan is formulated, the particles each having the transport process simulated include the neutrons and the photons.

For the step that the macroscopic cross-section is calculated according to the information of the particle:

The macroscopic cross-section of the particle is associated with the energy of the particle and a tissue material of a voxel grid of the particle. After the information of the particle is acquired, a material of a voxel grid of the particle is determined on the basis of the position information and the direction information of the particle. The macroscopic cross-section is calculated on the basis of the energy information of the particle and the material of the voxel grid of the particle.

For the step that the next transport point or the collision point of the particle is acquired on the basis of the macroscopic cross-section:

A collision probability is calculated on the basis of the macroscopic cross-section. The next transport point or the collision point of the particle is sampled. Since the position or the direction or the energy of the particle changes at the sampled next transport point or collision point of the particle, the information of the particle is to be updated.

For the step that the survival status of the particle is determined:

The particle in the transport process may die for collision, and may also move out of the spatial simulation range, such as the particle moves out of the 3D voxel model. In this case, there is no need to simulate the particle continuously. Only the transport process of the particle survival in the 3D voxel model is simulated.

After the next collision point or the transport point of the particle is sampled on the basis of the macroscopic cross-section, the survival status of the particle is to be determined.

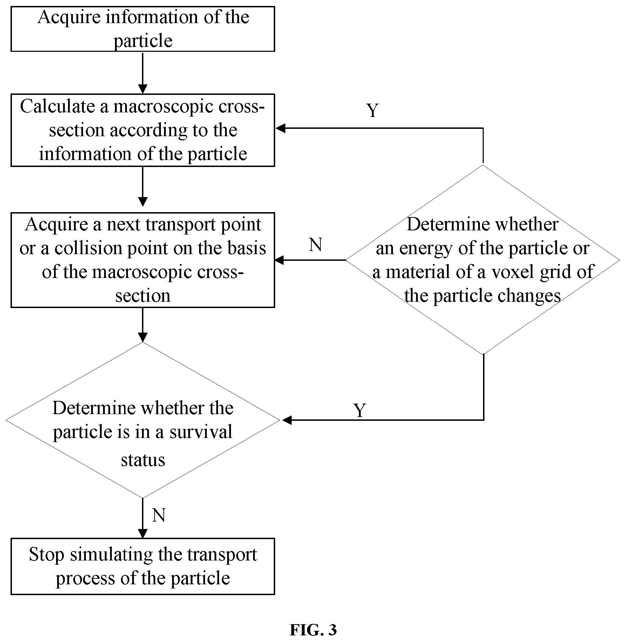

As shown in FIG. 3, in an optional implementation, if the particle is in a survival state, whether to recalculate the macroscopic cross-section is determined. If the particle is not in the survival state, simulating the transport process of the particle is stopped.

More specifically, the step that whether to recalculate the macroscopic cross-section is determined at least includes: Whether the energy of the particle changes is determined. Whether the material of the voxel grid of the particle changes is determined. If the energy of the particle or the material of the voxel grid of the particle changes, the macroscopic cross-section is recalculated according to the energy of the particle and the material of the voxel grid of the particle. If the energy of the particle and the material of the voxel grid of the particle do not change, the next transport point or the collision point of the particle is acquired on the basis of the macroscopic cross-section. When both the material of the voxel grid of the particle and the energy of the particle do not change, the macroscopic cross-section in the previous step can be used, and the next transport point or the collision point of the particle is acquired directly on the basis of the macroscopic cross-section. This reduces a number of times for calculating the macroscopic cross-section, thereby reducing the operation time.

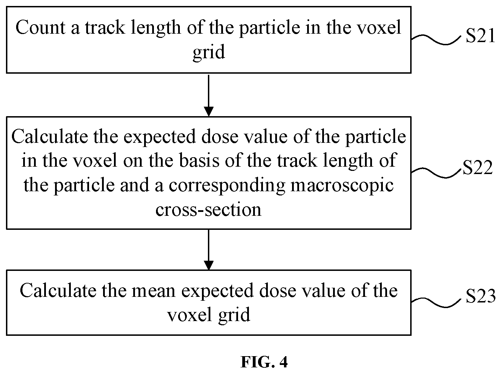

As shown in FIG. 4, in the method for optimizing Monte Carlo calculation provided by the present disclosure, the dose counting method at least includes:

A track length of the particle in the voxel grid is counted. The expected dose value of the particle in the voxel grid is calculated on the basis of the track length of the particle and a corresponding macroscopic cross-section. The mean expected dose value of the voxel grid is calculated.

In the Monte Carlo method, the dose is usually counted with the track length. The particle leaves a track for each movement in the grid. According to the track length and the macroscopic cross-section, the collision probability of the particle in the grid can be calculated. By multiplying the collision probability by mean energy deposition of the particle in each collision, energy deposition on the track can be obtained. Energy deposition of all particles in the grid is accumulated, and then normalized to obtain the expected dose value of the grid. According to the conventional dose counting method, a track of each particle is simulated. A track length of the particle and a serial number of each of grids that the particle passes through are respectively recorded with two arrays. An expected dose value of the grid is calculated according to the track length as well as a macroscopic cross-section. If the particle passes through the grid repeatedly, the array for recording the serial number of the grid includes a same serial number. The expected dose values at the same serial number are merged. At last, the expected dose values of the particle are accumulated to a total dose. Due to a large number of grids that the particle passes through, it is time-consuming to merge the expected dose values of the same grid. The dose counting method provided by the present disclosure omits the process for merging doses of the same particle passing twice through the same voxel grid, thereby greatly saving the dose calculation time. For the step that the mean expected dose value of the voxel grid is calculated:

The mean expected dose value of the voxel grid is calculated by:

$$mean = \frac{\sum_{i=1}^{N} t_i}{N} \qquad (1)$$

where, i is a serial number of the particle, $t_i$ is an expected dose value of the particle i in the voxel grid, and N is a total number of particles.

When the particle passes twice through the voxel grid, the expected dose value of the particle is counted twice, and the total number N of particles is unchanged. By omitting the process for merging the expected dose values of the same particle passing twice through the voxel grid, directly taking two individual particles for calculation, and keeping the total number N of particles unchanged, the method ensures the accuracy of the calculated dose result, and reduces the time for the dose calculation, thereby reducing the operation time. Assuming that the ith particle passes twice through the grid, and the expected dose values are t1 and t2, ti=t1+t2. If the doses of the same particle are not merged, but two individual particles are used for calculation, the ti is still unchanged. Without affecting the accuracy of the dose calculation, this effectively saves the time of the dose calculation.

In other optional implementation, the dose counting method further at least includes: A dose error of the voxel grid is calculated.

Further, the step that the dose error of the voxel grid is calculated includes: The dose error of the voxel grid is calculated by:

$$error = \sqrt{\frac{\frac{\sum_{i=1}^{N} t_i^2}{N} - mean^2}{N-1}} \qquad (2)$$

where, i is a serial number of the particle, $t_i$ is an expected dose value of the particle i in the voxel grid, and N is a total number of particles.

When the particle passes twice through the voxel grid, the expected dose value of the particle is counted twice, and the total number N of particles is unchanged.

Another aspect of the present disclosure provides a neutron capture therapy system, including: an image acquisition module configured to acquire a medical image of an irradiated body; and a treatment planning module configured to establish a 3D voxel model on the basis of the medical image and formulate a treatment plan. The treatment planning module includes at least a transport calculation unit and a dose counting unit. The transport calculation unit is configured to simulate a transport process of a particle. The dose counting unit is configured to count an expected dose value of the particle, and calculate a mean expected dose value of each of voxel grids on the basis of the expected dose value of the particle.

In other optional implementations, the neutron capture therapy system further includes a dose calculation unit. The dose calculation unit is configured to calculate a tissue dose value on the basis of the mean expected dose value of the voxel grid. The treatment planning module is further configured to formulate the treatment plan on the basis of the tissue dose value.

According to the neutron capture therapy system provided by the present disclosure, by optimally simulating the transport process or the dose counting method of the particle, the Monte Carlo calculation is optimized, thereby shortening time for formulating the treatment plan.

The illustrative specific implementations of the present disclosure are described above to facilitate those skilled in the art to understand the present disclosure, but it should be noted that the present disclosure is not limited to the scope of the specific implementations. Various obvious changes made by those of ordinary skill in the art within the spirit and scope of the present disclosure defined by the appended claims should fall within the protection scope of the present disclosure.

**Claims**

1. A method for optimizing Monte Carlo calculation, at least comprising:

   simulating a transport process of a particle with a transport calculation method;
   counting an expected dose value of the particle with a dose counting method; and
   calculating a mean expected dose value of each of voxel grids on the basis of the expected dose value of the particle.

2. The method for optimizing Monte Carlo calculation according to claim 1, wherein the transport calculation method at least comprises:

   acquiring information of the particle;
   calculating a macroscopic cross-section according to the information of the particle;
   acquiring a next transport point or a collision point of the particle on the basis of the macroscopic cross-section; and
   determining a survival status of the particle.

3. The method for optimizing Monte Carlo calculation according to claim 2, wherein the acquiring a next transport point or a collision point of the particle on the basis of the macroscopic cross-section comprises:
   sampling the next transport point or the collision point of the particle, and updating the information of the particle.

4. The method for optimizing Monte Carlo calculation according to claim 2, wherein the determining a survival status of the particle at least comprises:

   if the particle is in a survival state, determining whether to recalculate the macroscopic cross-section; and
   if the particle is not in the survival state, stopping simulating the transport process of the particle.

5. The method for optimizing Monte Carlo calculation according to claim 4, wherein the information of the particle at least comprises: position information, direction information, and energy information of the particle.

6. The method for optimizing Monte Carlo calculation according to claim 5, further comprising: determining a material of a voxel grid of the particle on the basis of the position information and the direction information of the particle, and calculating the macroscopic cross-section on the basis of the energy information of the particle and the material of the voxel grid of the particle.

7. The method for optimizing Monte Carlo calculation according to claim 6, wherein the determining whether to recalculate the macroscopic cross-section at least comprises:

   determining whether the energy of the particle changes;
   determining whether the material of the voxel grid of the particle changes;
   if the energy of the particle or the material of the voxel grid of the particle changes, recalculating the macroscopic cross-section according to the energy of the particle and the material of the voxel grid of the particle; and
   if the energy of the particle and the material of the voxel grid of the particle do not change, acquiring the next transport point or the collision point of the particle on the basis of the macroscopic cross-section.

8. The method for optimizing Monte Carlo calculation according to claim 1, wherein the dose counting method at least comprises:

   counting a track length of the particle in the voxel grid;
   calculating the expected dose value of the particle in the voxel grid on the basis of the track length of the particle and a corresponding macroscopic cross-section; and
   calculating the mean expected dose value of the voxel grid.

9. The method for optimizing Monte Carlo calculation according to claim 8, wherein the calculating the mean expected dose value of the voxel grid comprises:

    calculating the mean expected dose value of the voxel grid by:

$$mean = \frac{\sum_{i=1}^{N} t_i}{N} \qquad (1)$$

    wherein, i is a serial number of the particle, $t_i$ is an expected dose value of the particle i in the voxel grid, and N is a total number of particles.

10. The method for optimizing Monte Carlo calculation according to claim 9, wherein the calculating the mean expected dose value of the voxel grid further comprises:
    when the particle passes twice through the voxel grid, counting the expected dose value of the particle twice, and keeping the total number N of particles unchanged.

11. The method for optimizing Monte Carlo calculation according to claim 8, wherein the dose counting method further comprises:

    calculating a dose error of the voxel grid, comprising: calculating the dose error of the voxel grid by:

$$error = \sqrt{\frac{\frac{\sum_{i=1}^{N} t_i^2}{N} - mean^2}{N-1}} \qquad (2)$$

    wherein, i is a serial number of the particle, $t_i$ is an expected dose value of the particle i in the voxel grid, and N is a total number of particles.

12. The method for optimizing Monte Carlo calculation according to claim 11, wherein the calculating the dose error of the voxel grid further comprises:
    when the particle passes twice through the voxel grid, counting the expected dose value of the particle twice, and keeping the total number N of the particles unchanged.

13. The method for optimizing Monte Carlo calculation according to claim 1, wherein the particles comprise a neutron and a photon.

14. A neutron capture therapy system, comprising:

    an image acquisition module configured to acquire a medical image of an irradiated body; and
    a treatment planning module configured to establish a three-dimensional (3D) voxel model on the basis of the medical image and formulate a treatment plan,
    wherein the treatment planning module comprises at least a transport calculation unit and a dose counting unit, the transport calculation unit is configured to simulate a transport process of a particle, and the dose counting unit is configured to count an expected dose value of the particle, and calculate a mean expected dose value of each of voxel grids on the basis of the expected dose value of the particle.

15. The neutron capture therapy system according to claim 14, further comprising a dose calculation unit, wherein the dose calculation unit is configured to calculate a tissue dose value on the basis of the mean expected dose value of the voxel grid; and the treatment planning module is further configured to formulate the treatment plan on the basis of the tissue dose value.

Simulate a transport process of each of particles — S10

Count an expected dose value of the particle — S11

Calculate a mean expected dose value of each of voxel grids — S12

**FIG. 1**

Acquire information of the particle — S11

Calculate a macroscopic cross-section according to the information of the particle — S12

Acquire a next transport point or a collision point on the basis of the macroscopic cross-section — S13

Determine whether the particle is in a survival status — S14

**FIG. 2**

Acquire information of the
particle

↓

Calculate a macroscopic cross-
section according to the
information of the particle

Y

↓

Acquire a next transport point
or a collision point on the basis
of the macroscopic cross-
section

N

Determine whether
an energy of the particle or
a material of a voxel grid of
the particle changes

↓

Determine whether the
particle is in a survival
status

Y

N

Stop simulating the transport
process of the particle

**FIG. 3**

| Count a track length of the particle in the voxel grid | S21 |

↓

| Calculate the expected dose value of the particle in the voxel on the basis of the track length of the particle and a corresponding macroscopic cross-section | S22 |

↓

| Calculate the mean expected dose value of the voxel grid | S23 |

**FIG. 4**

# INTERNATIONAL SEARCH REPORT

| International application No. |
| --- |
| **PCT/CN2023/134368** |

| A. | CLASSIFICATION OF SUBJECT MATTER |
| --- | --- |

A61N 5/10(2006.01)i

According to International Patent Classification (IPC) or to both national classification and IPC

| B. | FIELDS SEARCHED |
| --- | --- |

Minimum documentation searched (classification system followed by classification symbols)

IPC:A61N

Documentation searched other than minimum documentation to the extent that such documents are included in the fields searched

Electronic data base consulted during the international search (name of data base and, where practicable, search terms used)

CNTXT, WPABS, ENTXTC, CNKI: 蒙特卡罗, 计算, 优化, 模拟, 粒子, 剂量, 统计, 期望, 宏观, 截面, 值, 存活, 能量, 变化, 径迹, 长度, 体素, 网格, 误差, Monte, Carlo, calculation, optimize, simulate, particle, dose, statistic, expectation, macro, cross-section, value, survival, energy, variation, track, length, voxel, grid, error

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 114681816 A (NEUBORON THERAPY SYSTEM LTD.) 01 July 2022 (2022-07-01) description, paragraphs 2-102, and figures 1-8 | 1-8, 13-15 |
| A | CN 104346533 A (HEFEI INSTITUTES OF PHYSICAL SCIENCE, CHINESE ACADEMY OF SCIENCES) 11 February 2015 (2015-02-11) entire document | 1-15 |
| A | CN 106355017 A (HEFEI INSTITUTES OF PHYSICAL SCIENCE, CHINESE ACADEMY OF SCIENCES) 25 January 2017 (2017-01-25) entire document | 1-15 |
| A | CN 110504016 A (LINKINGMED TECHNOLOGY CO., LTD.) 26 November 2019 (2019-11-26) entire document | 1-15 |
| A | CN 110675932 A (LINKINGMED TECHNOLOGY CO., LTD.) 10 January 2020 (2020-01-10) entire document | 1-15 |

☑ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **20 December 2023** | **23 December 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/134368**

| C. | DOCUMENTS CONSIDERED TO BE RELEVANT | | |
|---|---|---|
| Category* | Citation of document, with indication, where appropriate, of the relevant passages | Relevant to claim No. |
| A | CN 111111018 A (23 UNIT, UNIT 96901 OF PLA) 08 May 2020 (2020-05-08) entire document | 1-15 |
| A | US 2017304651 A1 (HITACHI, LTD.) 26 October 2017 (2017-10-26) entire document | 1-15 |
| A | US 6148272 A (UNIVERSITY OF CALIFORNIA) 14 November 2000 (2000-11-14) entire document | 1-15 |

Form PCT/ISA/210 (second sheet) (July 2022)

| | INTERNATIONAL SEARCH REPORT | International application No. |
| | Information on patent family members | PCT/CN2023/134368 |

| Patent document cited in search report | | | Publication date (day/month/year) | Patent family member(s) | | | Publication date (day/month/year) |
|---|---|---|---|---|---|---|---|
| CN | 114681816 | A | 01 July 2022 | TW | 202325359 | A | 01 July 2023 |
| | | | | TW | 202326749 | A | 01 July 2023 |
| | | | | US | 2023330433 | A1 | 19 October 2023 |
| | | | | EP | 4272813 | A1 | 08 November 2023 |
| | | | | TW | 202326750 | A | 01 July 2023 |
| | | | | TW | 202326748 | A | 01 July 2023 |
| | | | | WO | 2022143333 | A1 | 07 July 2022 |
| | | | | TW | 202228147 | A | 16 July 2022 |
| | | | | TWI | 800177 | B | 21 April 2023 |
| CN | 104346533 | A | 11 February 2015 | None | | | |
| CN | 106355017 | A | 25 January 2017 | None | | | |
| CN | 110504016 | A | 26 November 2019 | None | | | |
| CN | 110675932 | A | 10 January 2020 | None | | | |
| CN | 111111018 | A | 08 May 2020 | None | | | |
| US | 2017304651 | A1 | 26 October 2017 | US | 10376713 | B2 | 13 August 2019 |
| | | | | WO | 2016047194 | A1 | 31 March 2016 |
| | | | | JPWO | 2016047194 | A1 | 06 July 2017 |
| | | | | JP | 6421194 | B2 | 07 November 2018 |
| US | 6148272 | A | 14 November 2000 | AU | 1605000 | A | 24 July 2000 |
| | | | | JP | 2003521278 | A | 15 July 2003 |
| | | | | JP | 3730514 | B2 | 05 January 2006 |
| | | | | DE | 69922994 | D1 | 03 February 2005 |
| | | | | DE | 69922994 | T2 | 15 December 2005 |
| | | | | ATE | 285820 | T1 | 15 January 2005 |
| | | | | ES | 2237183 | T3 | 16 July 2005 |
| | | | | EP | 1128873 | A1 | 05 September 2001 |
| | | | | EP | 1128873 | B1 | 29 December 2004 |
| | | | | CA | 2350439 | A1 | 13 July 2000 |
| | | | | CA | 2350439 | C | 02 August 2005 |
| | | | | WO | 0040298 | A1 | 13 July 2000 |

Form PCT/ISA/210 (patent family annex) (July 2022)